**Europäisches Patentamt**

**European Patent Office**

**Office européen des brevets**

(19)

(11) Numéro de publication: **0 124 158**
**B1**

(12) # FASCICULE DE BREVET EUROPÉEN

(45) Date de publication du fascicule du brevet:
**22.07.87**

(51) Int. Cl.⁴: **A 61 K 6/02**

(21) Numéro de dépôt: **84200462.4**

(22) Date de dépôt: **30.03.84**

(54) Procédé de traitement d'une surface dentaire.

(30) Priorité: **05.04.83 FR 8305534**

(43) Date de publication de la demande:
**07.11.84 Bulletin 84/45**

(45) Mention de la délivrance du brevet:
**22.07.87 Bulletin 87/30**

(84) Etats contractants désignés:
**BE DE FR IT LU NL**

(56) Documents cité:
**DE-A-2 050 089**
**FR-A-2 131 816**

**HAGERS HANDBUCH DER PHARMAZEUTISCHEN PRAXIS, vol. I, 4ème édition, 1967, Springer-Verlag, BERLIN (DE), page 356.**

(73) Titulaire: **LABORATOIRE S.P.A.D., F-21800 Quetigny (FR)**

(72) Inventeur: **Geissant, Jean- Louis, Dr., 15, Place de l'Hôtel- de- Ville, F-42130 Boen- sur- Lignon (FR)**

(74) Mandataire: **Silhol, Christian Maurice Alfred, c/o BUGNION S.A. Conseils en Propriété Industrielle 10, route de Florissant Case Postale 375, CH- 1211 Genève 12 - Champel (CH)**

EP 0 124 158 B1

LIBER, STOCKHOLM 1987

## Description

L'invention concerne un procédé de traitement d'une surface dentaire mécaniquement usinée et mordancée moyennant un agent acide, tel que de l'acide phosphorique dilué, précédant la pose de résines de reconstitution, et une solution neutralisante pour la mise en ouvre de ce procédé.

Le document DE-A-2 050 089 décrit, dans l'exemple 1, un procédé de traitement d'une surface dentaire où une solution neutralisante est appliquée sur cette surface après le mordançage et avant la pose de la reconstitution.

Une solution tampon à base de:
- une solution aqueuse contenant un acide aminé tel que le glycocolle;
- de la soude;
- du chlorure de sodium et présentant un pH d'env. 10,
est décrite par "HAGERS HANDBUCH DER PHARMAZEUTISCHEN PRAXIS" vol. (1967) page 356.

Il est connu que l'action de l'acide lors de mordançage se poursuit au-delà de rinçage et de la pose de composite des résines de reconstitution. Ainsi, la visualisation au microscope électronique à balayage d'une zone d'émail mordancé quinze jours auparavant, avec de l'acide phosphorique à 45 % durant une minute et demie, puis fracturé perpendiculairement lors de la séance de microscopie, montre dans tous les cas une déminéralisation typique de la substance interprismatique jusqu'à une profondeur variant de 40 à 50 µm selon les caractéristiques et la durée d'action de l'acide phosphorique et, dans certains cas, si l'acidité résiduelle n'est pas supprimée par un rinçage abondant, une déminéralisation anarchique en sous-surface de 60 µm de profondeur environ se produit.

Cette déminéralisation non souhaitée consécutive à l'utilisation de l'acide a pu être mise en évidence au microscope électronique à balayage par la présence de cristaux résultant d'une modification trop lente de la valeur pH lors du rinçage, entraînant de ce fait une précipitation de belles structures cristallines, par la visualisation d'une transformation progressive des prismes d'émail en BRUSHITE (phosphate dibasique de calcium dihydraté en cristaux monocliniques) et par la visualisation d'une zone poreuse qualifiée de "banc de sable" incapable de soutenir à long terme une reconstitution.

A la microsonde, elle se révèle par l'inversion des pics de calcium et de phosphore sur les cristaux de phosphate de calcium, par rapport aux prismes d'émail d'une dent non mordancée servant de témoin, par la diminution de la concentration en phosphore dans la zone mordancée d'une façon typique, et par l'égalisation relative des concentrations en phosphore et en calcium au niveau de la zone atypique. Ainsi l'analyse à la microsonde d'une zone d'émail mordancé avec $H_3PO_4$ de 45 % pendant 1 min 30, rincé puis fracturé et carboné, sur 90 µm de profondeur en comparaison avec un témoin d'apatite géologique (MP = analyse sur prime ; MSIP = analyse sur substance interprismatique) a montré:

Lors de l'analyse du témoin, la hauteur moyenne du pic d'énergie par le phosphore est de 42363,66 et de 52184 pour le calcium.

Pour l'émail mordancé, l'analyse des prismes (MP) et de la substance interprismatique (MSIP) montre un début d'inversion des pics d'énergie en surface, puis une perte progressive de Ca et P de 0 à 40 µm de profondeur. Enfin, de 40 à 90 µm, on observe, outre la normalisation du rapport phospho-calcique, une perte limitée de phosphore et de calcium à 50 microns, montrant une déminéralisation consécutive à une acidité résiduelle et prolongée.

C'est dans cette dernière zone de 50 à 60 µm de profondeur et sur un échantillon doré qu'on a pu mettre en évidence la transformation des prismes d'émail en BRUSHITE avec égalisation relative des pics de P et Ca sur les prismes d'émail et l'inversion vraie sur les baguettes de cristal ainsi formées. De plus c'est dans cette même zone que se propose d'agir le produit de l'invention, en neutralisant l'acide résiduel.

Il est à retenir de cette expérimentation que l'eau ne possède pas les qualités nécessaires et suffisantes pour neutraliser cette activité acide résiduelle en profondeur. C'est la raison pour laquelle le composite ainsi réalisé sur un tel terrain ne peut espérer une longévité comparable à celle d'un amalgame d'argent.

D'autre part, les colorations périphériques, notamment tabagiques, ne sont pas dues aux composites mêmes, mais à la mise à jour a posteriori de la strate d'émail ainsi altérée par l'acidité résiduelle.

Enfin, notamment dans les reconstitutions dites esthétiques et du fait de la présence de cette couche en sous-surface, les reconstitutions composites réalisées possèdent une moindre résistance à la traction. Ceci est surtout mis en évidence lors des décollements fréquents des prothéses d'orthodontie pendant des traitements exigeant l'application de forces importantes.

Le but l'invenion est d'eviter ces inconvénients par une neutralisation de l'acidité résiduelle après l'action sur l'émail dentaire et, en même temps, de favoriser l'ancrage des résines de reconstitution.

Le procédé employé pour résoudre ce probleme est, selon l'invention, caractérisé en ce qu'une solution aqueuse tamponnée et détergente, contentant un acide aminé, du chlorure de sodium, de la soude, ainsi que de l'alcool et un agent mouillant et présentant une valeur pH d'environ 10, est appliquée sur cette surface dentaire après le mordançage et/ou avant la pose de la reconstitution.

Une telle solution s'utilise après rinçage et séchage grossier de la surface dentaire mordancée durant quelques secondes, suivis d'un rinçage et d'un séchage abondants.

Toutefois, une bonne partie de l'acide phosphorique ayant réagi avec l'émail, il ne reste qu'une fonction résiduelle à neutraliser. Malheureusement on ne peut pas connaître avec précision la quantité ayant réagi. Il n'est donc pas opportun d'utiliser une base forte.

La solution neutralisante et tamponnée pour la mise en oeuvre d'un tel procédé est, selon une autre particularité de l'invention, caractérisée en ce qu'elle consiste en une solution aqueuse contenant un acide aminé, de la soude et du chlorure de sodium et présentant une valeur pH d'environ 10, donc peu agressive, de l'alcool et un agent mouillant. L'alcool étant un déshydratant, facilite le rinçage et le séchage. Le mouillant quant à lui favorise le nettoyage des microcavités créées et assure une augmentation de la pénétration de cette solution neutralisante détergente dans toutes les zones mordancées. En outre, l'alcool ainsi que l'agent mouillant favorisent aussi l'élimination du phosphate de sodium éventuellement formé.

Le phosphate de sodium ainsi formé est éliminé lors du rinçage ultérieur. Dans le cas contraire le métabolisme phospho-calcique propre à l'organe dentaire récupère ce produit de réaction. Ce dernier phénomène est non agressif au niveau pulpaire.

Dans une exécution préférée, une solution neutralisante, tamponnée et détergente selon l'invention consiste en

100 ml d'eau purifiée
6,57 g de glycocolle
5,12 g de chlorure de sodium
60 ml 1 n-solution de soude
1,5 g d'un agent mouillant
33 ml d'alcool 95°

et présente une valeur pH de 9,75.

Comme agent mouillant on peut utiliser par exemple le polysorbate 20.

Théoriquement, un volume de cette solution neutralise exactement un même volume mordanceur ayant déjà réagi sur la dentine dans l'hypothèse où la quantité d'acide phosphorique n'ayant pas réagi représente 6,55 % de la quantité initiale mise en oeuvre. Ceci implique l'utilisation d'acide phosphorique en excès.

En tout cas, une telle solution neutralisante supprime les fondations suspectes résultant de la mauvaise neutralisation de l'acide phosphorique utilisé, par l'eau pas assez mouillante et non neutralisante.

Elle augmente la résistance à la traction de 20 à 40 % selon la méthodologie propre à chaque praticien, d'où son avantage dans la pose de prothéses en traitement orthodontique et dans la pose d'une reconstitution composite esthétique sur cavité peu ou pas rétentive.

Elle supprime l'apparition d'une coloration marginale d'origine dentaire due à l'inter-face et à la strate mal neutralisée.

Elle reste biologiquement non agressive pour les tissus dentaires et pour la pulpe, d'une part par suppression de l'acidité résiduelle et, d'autre part, par l'action non toxique du phosphate de sodium récupéré, le cas échéant, par le métabolisme phosphocalcique dentaire.

Enfin elle augmente la longévité des reconstitutions composites utilisant la méthode de moidançage suivie d'application de résine fluide.

En effet, l'utilisation d'une telle solution neutralisante de l'activité acide résiduelle, après l'action sur la dent d'un mordanceur, constitue une nouvelle méthode se décomposant ainsi: mordancer la surface de l'émail dentaire concernée par une solution de H3 PO4 de 45 % = 4,58 molaire durant 1,5 min, rinçage et séchage grossiers, application de la solution neutralisante durant quelques secondes, rinçage et séchage abondants et application de résine fluide et de la résine de reconstitution à l'abri de l'humidité et sous pression.

## Revendications

1. Procédé de traitement d'une surface dentaire mécaniquement usinée et mordancée au moyen d'un agent acide précédant la pose des résines de reconstitution caractérisé en ce qu'une solution aqueuse tamponnée et détergente, contenant un acide aminé, du chlorure de sodium et de la soude ainsi que de l'alcool et un agent mouillant, et présentant une valeur pH d'environ 10, est appliquée.

2. Procédé selon la revendication 1 caracterisé en ce que la solution est appliquée durant quelques secondes suivie d'un rinçage et séchage.

3. Solution tamponnée pour le traitement d'une surface dentaire mécaniquement usinée et mordancée au moyen d'un agent acide précédant la pose des résines de reconstitution caractérisée en ce qu'elle consiste en une solution aqueuse contentant un acide aminé, du chlorure de sodium et de la soude ainsi que de l'alcool et un agent mouillant et présentant une valeur pH d'env. 10.

4. Solution selon la revendication 3, caractérisée en ce qu'elle consiste en

100 ml d'eau purifiée
6,57 g de glycocolle
5,12 g de chlorure de sodium
60 ml 1 n-solution de soude
1,5 g d'un agent mouillant
33 ml d'alcool 95°

et présente une valeur pH de 9,75.

5. Solution selon la revendication 4, caractérisée en ce que la polysorbate 20 est utilisé comme agent mouillant.

## Patentansprüche

1. Verfahren zur Behandlung einer Zahnoberfläche, welche mechanisch bearbeitet und mittels eines sauren Mittel, gebeizt wurde vor dem Auftragen von Erneuerungsharzen, dadurch gekennzeichnet, dass eine wässrige, gepufferte und entspannte Lösung, welche eine Aminosäure, Natriumchlorid und Soda sowie Alkohol und ein Netzmittel enthält und einen pH-Wert von etwa 10 aufweist, aufgebracht wird.

2. Verfahren nach Anspruch 1, dadurch gekennzeichnet, dass die Lösung wahrend einiger Sekunden, gefolgt von einer Spülung und Trocknung aufgebracht wird.

3. Gepufferte Lösung für die Behandlung einer Zahnoberfläche, welche mechanisch bearbeitet und mittels eine, sauren Mittels gebeizt wurde vor dem Auftragen von Erneuerungsharzen, dadurch gekennzeichnet, dass sie aus einer wässrigen Lösung besteht, welche eine Aminosäure, Natriumchlorid und Soda sowie Alkohol und ein Netzmittel enthält und einen pH-Wert von etwa 10 aufweist.

4. Lösung nach Anspruch 3, dadurch gekennzeichnet, dass sie aus
   100 ml gereinigtem Wasser
   6,57 g Glykokoll
   5,12 g Natriumchlorid
   60 ml 1n-Sodalösung
   1,5 g eines Netzmittels
   33 ml Alkohol 95°
   besteht und einen PH-Wert von 9,75 aufweist

5. Lösung nach Anspruch 4, dadurch gekennzeichnet, dass Polysorbat 20 als Netzmittel verwendet wird.

## Claims

1. Method of treating mechanically machined and mordanted dental surfaces by means of an acid agent preliminarily to the fitting of reconstitution resins, characterised in that an aqueous, buffered and detergent solution containing an amino acid, sodium chloride and sodium hydroxide as well as alcohol and a wetting agent, and having a pH value of about 10, is applied.

2. The method of Claim 1, characterised in that the solution is applied during a few seconds and followed by rinsing and drying steps.

3. A buffered solution for treating a mechanically machined and mordanted dental surface by means of an acid agent preliminarily to the fitting of reconstitution resins, characterised in that it consists of an aqueous solution containing an amino acid, sodium chloride and sodium hydroxide, as well as alcohol and a wetting agent, and having a pH value of about 10.

4. A solution according to Claim 3, characterised in that it consists of:
   100 ml of purified water
   6.57 g of glycocoll
   5.12 g of sodium chloride
   60 ml of 1 n- sodium hydroxide
   1,5 g of a wetting agent
   33 ml of 95° alcohol,

and having a pH value of 9,75.

5. The solution of Claim 4, characterised in that the wetting agent consists of polyscorbate 20.